# EUROPEAN PATENT APPLICATION

(11) **EP 4 190 377 A1**
(43) Date of publication of application: **07.06.2023**
(21) Application number: 21858340.9
(22) Date of filing: 18.08.2021
(51) Int. Cl.: A61M 5/31, A61M 5/315, A61M 5/50

(54) **PRE-FILLED SYRINGE**

(30) Priority: 20.08.2020 JP 2020139686
(71) Applicant: TERUMO Kabushiki Kaisha, Tokyo 151-0072 (JP)
(72) Inventor: TONO Yohei, Nakakoma-gun, Yamanashi 409-3853 (JP); SHIMIZU Makoto, Yamaguchi-shi, Yamaguchi 754-0894 (JP)
(74) Representative: Casalonga
(86) International application number: PCT/JP2021/030194
(87) International publication number: WO 2022/039196

(57) **Abstract**

A prefilled syringe 1 according to the present invention includes an outer cylinder 2, a gasket 3, a plunger 4, and a drug 9 housed in the outer cylinder 2. The outer cylinder 2 includes a flange part 6 that is located at a rear end part. The plunger 4 includes a gasket pressing part 43 and a shaft part 41 that extends in a rear end direction from a gasket pressing part 43. The shaft part includes indicator parts 45a, 45b, 45c, and 45d that are located on a rear end side of the gasket pressing part and can be recognized by visually checking an external appearance, and indicator parts are located in the flange part 6 of the outer cylinder 2 in a state where the plunger has been attached to the gasket 3.

## Description

### Technical Field

The present invention relates to a prefilled syringe in which a plunger is attached to a gasket.

### Background Art

A drug is housed in a sterile state inside a prefilled syringe in advance. Then, the prefilled syringe punctures a patient as it is, injects the drug into a medical container, or is attached to a syringe pump to administer the drug to the patient.

There are a prefilled syringe of a type in which a plunger is not attached to a gasket and is attached at a time of use, and a prefilled syringe of a type in which a plunger is attached to a gasket in advance.

According to the prefilled syringe of the type in which the plunger is attached to the gasket, a method that is so-called vacuum capping of filling a drug in an outer cylinder whose distal end opening is sealed in a sterile environment and under a depressurized atmosphere (a vacuum atmosphere), disposing a gasket at an opening part of the outer cylinder, then producing a normal pressure state, and thereby inserting the gasket into the outer cylinder is used to insert the gasket. Then, the plunger is attached to the gasket to manufacture the prefilled syringe.

The prefilled syringe in which a plunger has already been attached to a gasket makes it difficult to dispose the gasket at an opening part of an outer cylinder, and therefore the above-described capping work is performed using the gasket alone. Therefore, a prefilled syringe generally requires subsequent plunger attachment work.

Furthermore, a label that describes a drug name, a drug amount, and the like is generally pasted on an outer surface of an outer cylinder of a prefilled syringe, and the label often makes it impossible to visually check a gasket position.

Patent Literature 1 (JP 08-504354 W) discloses a prefilled syringe. The prefilled syringe according to Patent Literature 1 uses a plunger illustrated in Figs. 3, 8, and 10. The illustrated plunger includes four disc-shaped parts that can be inserted into an outer cylinder.

### Citation List

### Patent Literature

Patent Literature 1: JP 08-504354 W (WO94-013340, USP5370621, and USP5472431)

### Summary of Invention

### Technical Problem

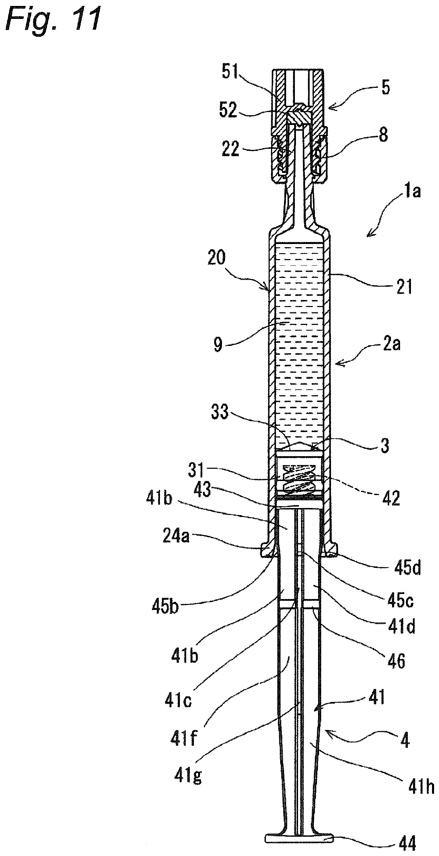

In the prefilled syringe according to Patent Literature 1, the plunger includes the four disc-shaped parts around a longitudinal axis, and the three disc-shaped parts are located in the outer cylinder in Fig. 11.

In a case where, for example, the third disc-shaped part is exposed from the outer cylinder in a state illustrated in Fig. 11, the gasket has moved backward from a state where the gasket is positioned in the syringe. However, Cited Literature 1 does not recognize such a case, and there is a probability that, even in a case where the third disc-shaped part is not exposed from the inside of a syringe main body, the gasket has moved backward from the state where the gasket is positioned in the syringe. Furthermore, the four disc-shaped parts have the same shape, and, even in a case where the third disc-shaped part is exposed from the outer cylinder, a user cannot recognize based on the exposure of the third disc-shaped part that the gasket has moved backward from the state where the gasket is positioned in the syringe.

Furthermore, the gasket is likely to move backward before use. This is considered to be mainly due to a user's unnecessary operation of moving the plunger backward immediately before administration. Furthermore, backward movement of the plunger (backward movement of the gasket) is also likely to occur due to deviation from environmental conditions such as a temperature environment at a time of manufacturing, a time of transportation, and a time of storage of the prefilled syringe.

When the plunger moves backward, in other words, the gasket moves backward beyond a certain level, a negative pressure may be generated in the syringe, and air may flow into a drug solution of the prefilled syringe from between the gasket and the outer cylinder, and thereby contaminate the drug solution.

An object of the present invention is to provide a prefilled syringe in which a plunger has been attached to a gasket, and that makes it possible to easily and reliably visually check that the plunger moves backward beyond an allowable level.

### Solution to Problem

What achieves the above object is as follows.

A prefilled syringe includes: an outer cylinder; a gasket that is slidably housed in the outer cylinder; a plunger that presses the gasket; a sealing member that seals a distal end part of the outer cylinder; and a drug that is housed in the outer cylinder, the outer cylinder includes a flange part that is provided at a rear end part, the gasket includes a plunger distal end part attachment part, the plunger includes a gasket pressing part that is provided at a distal end of the plunger, a shaft part that extends in a rear end direction from the gasket pressing part, and a pressing operation part that is provided at a rear end part of the shaft part, the shaft part includes on a rear end side of the gasket pressing part an indicator part that can be recognized by visually checking an external appearance, the plunger is attached to the gasket, and the indicator part is located in the flange part of the outer cylinder, and cannot be recognized by visually checking the external appearance in a state where the plunger has been attached to the gasket, and is exposed behind the flange part, and can be recognized by visually checking the external appearance when the plunger moves backward.

### Brief Description of Drawings

Fig. 1 is a front view of a prefilled syringe according to an embodiment of the present invention.
Fig. 2 is a partial cross-sectional view taken along line A-A in Fig. 1.
Fig. 3 is a back view of the prefilled syringe illustrated in Fig. 1.
Fig. 4 is a perspective view illustrating the prefilled syringe illustrated in Fig. 1 from a lower end side.
Fig. 5 is a front view of a plunger used in the prefilled syringe illustrated in Fig. 1.
Fig. 6 is a cross-sectional view taken along line B-B in Fig. 5.
Fig. 7 is a perspective view of the plunger illustrated in Fig. 5.
Fig. 8 is a longitudinal cross-sectional view of a gasket used in the prefilled syringe illustrated in Fig. 1.
Fig. 9 is an explanatory view of an outer cylinder used in the prefilled syringe illustrated in Fig. 1.
Fig. 10 is a front view of a prefilled syringe according to another embodiment of the present invention.
Fig. 11 is a partial cross-sectional view taken along line C-C in Fig. 10.
Fig. 12 is a front view of a plunger according to the another embodiment used in the prefilled syringe of the present invention.
Fig. 13 is a front view of the plunger according to another example used in the prefilled syringe of the present invention.
Fig. 14 is a front view of the plunger according to the another example used in the prefilled syringe of the present invention.
Fig. 15 is a front view of the plunger according to the another example used in the prefilled syringe of the present invention.
Fig. 16 is a front view of the plunger according to the another embodiment used in the prefilled syringe of the present invention.
Fig. 17 is a front view of the plunger according to the another example used in the prefilled syringe of the present invention.
Fig. 18 is a right side view of the plunger illustrated in Fig. 5.
Fig. 19 is a plan view of the plunger illustrated in Fig. 5.

### Description of Embodiments

Hereinafter, a prefilled syringe will be described using embodiments illustrated in the drawings.

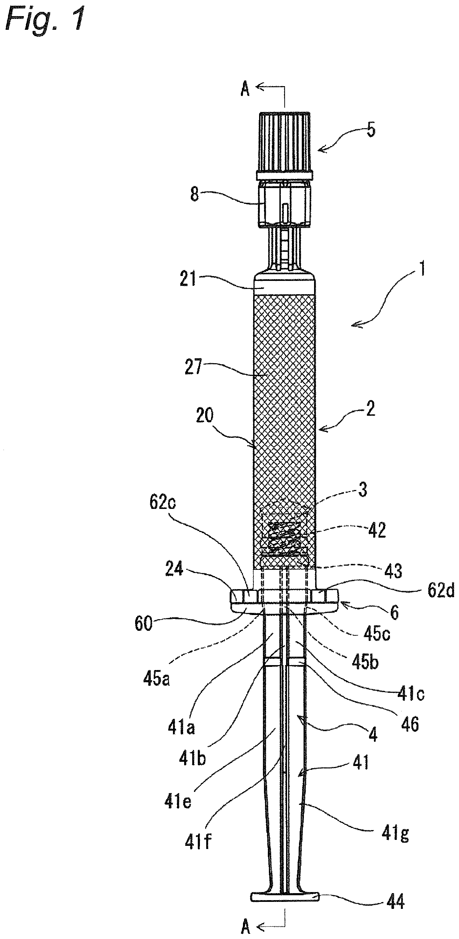

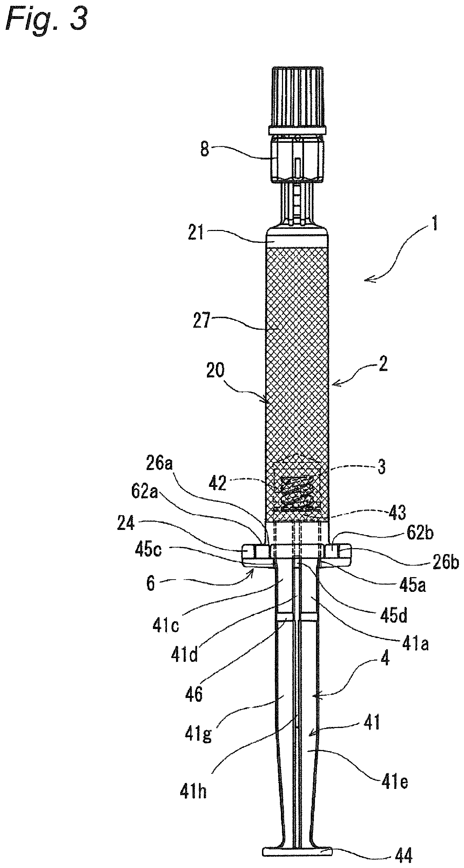

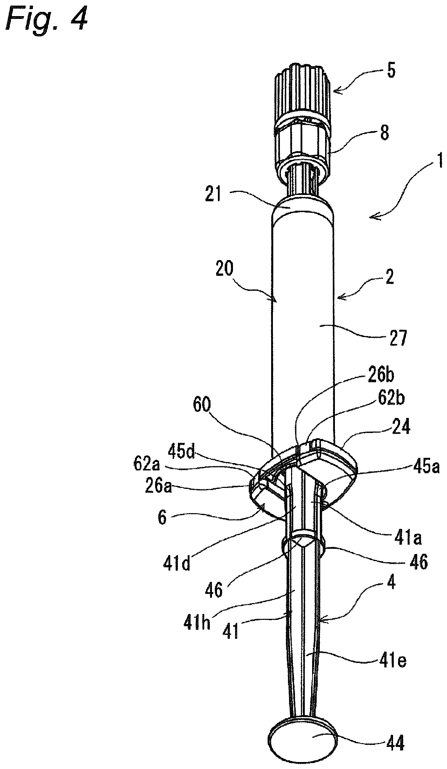

As illustrated in Figs. 1 to 4, a prefilled syringe 1 according to the present invention includes an outer cylinder 2, a gasket 3 that is slidably housed in the outer cylinder 2, a plunger 4 that presses the gasket 3, a sealing member 5 that seals a distal end part of the outer cylinder 2, and a drug 9 that is housed in the outer cylinder 2.

The outer cylinder 2 includes a flange part 6 that is located at a rear end part, the gasket 3 includes a plunger distal end part attachment part 35, and the plunger 4 includes a gasket attachment distal end part 42, a gasket pressing part 43 that is provided at a rear end of the gasket attachment distal end part 42, a shaft part 41 that extends in a rear end direction from the gasket pressing part 43, and a pressing operation part 44 that is provided at a rear end part of the shaft part 41.

The shaft part includes at a rear end side of the gasket pressing part 43 indicator parts 45a, 45b, 45c, and 45d that can be recognized by visually checking an external appearance, the plunger 4 is attached to the gasket 3, and the indicator parts 45a, 45b, 45c, and 45d are located in the flange part 6 of the outer cylinder 2, and cannot be recognized by visually checking the external appearance in a state where the plunger 4 has been attached to the gasket 3, and are exposed behind the flange part, and can be recognized by visually checking the external appearance when the plunger moves backward.

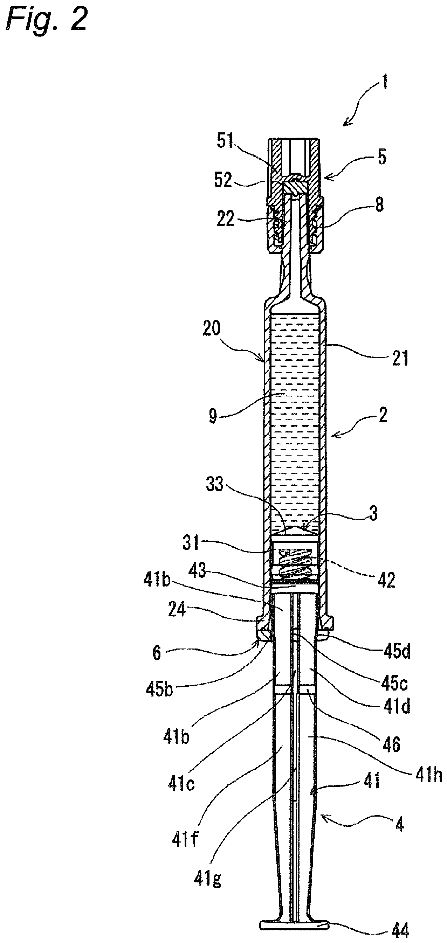

As illustrated in Figs. 1 to 4, the prefilled syringe 1 according to the present invention includes the outer cylinder 2, the gasket 3 that is slidably housed in the outer cylinder 2, the plunger 4 that presses the gasket 3, the sealing member 5 that seals the distal end part of the outer cylinder 2, and the drug 9 that is housed in the outer cylinder 2. In addition, Fig. 2 illustrates cross sections of the outer cylinder and the sealing member taken along line A-A in Fig. 1, and illustrates external appearances of the plunger and the gasket.

In the prefilled syringe 1 according to this embodiment, the plunger 4 can be attached to the gasket 3 by being rotated, and, when the plunger 4 is loosened from the gasket 3, the loosening can be resolved by rotating the plunger.

According to this embodiment, as illustrated in Figs. 1 to 4 and 9, the outer cylinder 2 includes an outer cylinder main body 20, and an attachment member 60 that is attached to a rear end part of the outer cylinder main body 20.

The outer cylinder main body 20 includes a hollow main body part 21, a hollow distal end part 22 that extends from a distal end part of the hollow main body part 21, and an outer cylinder flange 24 that is provided at a rear end part of the hollow main body part 21. In this embodiment, the attachment member 60 is fixed to the outer cylinder flange 24, and both of the attachment member 60 and the outer cylinder flange 24 form the flange part 6.

More specifically, the attachment member 60 is fixed to a bottom surface part of the flange 24 of the outer cylinder main body 20. The attachment member 60 is not limited to such a type, and may sandwich the flange 24.

In the prefilled syringe 1 according to this embodiment, the attachment member 60 includes retaining parts 64a and 64b that retains the plunger 4 in the outer cylinder 2. The plunger 4 includes a disc-shaped gasket pressing part 43, and this gasket pressing part 43 can abut on the retaining parts 64a and 64b of the attachment member 60, and has a function of retaining the plunger 4.

The outer cylinder main body 20 includes the hollow distal end part 22 that includes an opening at a distal end, a lock adapter 8 that covers a nozzle part of the hollow distal end part 22, the hollow main body part 21, and the flange 24 that is provided at a proximal end of the hollow main body part 21. The nozzle part can be connected with another medical instrument (e.g., an injection needle, a connector, or a three-way stopcock). The lock adapter 8 includes on an inner surface a screw part that can be screwed with medical instrument (e.g., a rib or a screw part formed on a flange of an injection needle, a connector, a three-way stopcock, or the like) connected to the nozzle part.

The hollow main body part 21 of the outer cylinder main body 20 is formed in a cylindrical shape having a relatively small diameter, and is filled with a small amount (more specifically, 0.5 ml to 2 ml) of a drug solution or the like in a first embodiment, and the outer diameter of the hollow main body part 21 is 6 to 12 mm.

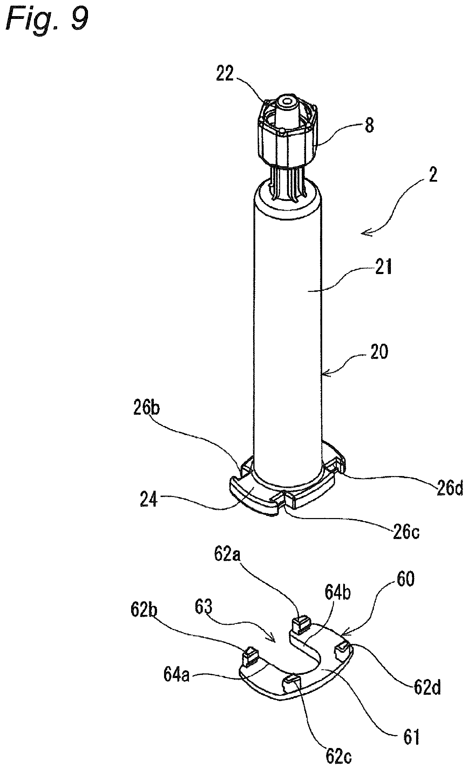

The outer cylinder flange 24 includes an arc-shaped outer edge that is formed protruding in a direction perpendicular to a center axis of the hollow main body part from an entire circumference of the proximal end of the hollow main body part 21. Furthermore, as illustrated in Fig. 9, the flange 24 includes four recess parts 26a, 26b, 26c, and 26d. Furthermore, as illustrated in Fig. 9, the attachment member 60 includes four protrusions 62a, 62b, 62c, and 62d that are provided on an upper surface of a plate-shaped part 61 and fit to the four recess parts of the flange 24. Furthermore, the attachment member 60 is attached to the flange 24 of the outer cylinder main body 20 by fitting the above protrusions and recess parts.

Furthermore, the attachment member 60 includes a plunger insertion cutout part 63 that extends in a center direction from an outer part, and the width of the plunger insertion cutout part 63 is smaller than an outer diameter of the gasket pressing part 43 of the plunger 4. Furthermore, the retaining parts 64a and 64b are formed by facing inner edge parts of the plunger insertion cutout part 63. Therefore, when the plunger 4 moves backward, the gasket pressing part 43 abuts on the retaining parts 64a and 64b, and is regulated from moving backward more and being detached from the outer cylinder.

The hollow distal end part 22 has a diameter that is smaller than the outer diameter of the hollow main body part 21, and is reduced toward the distal end. The hollow distal end part 22 includes at a distal end a distal end opening part for discharging a drug solution or the like in the outer cylinder. Furthermore, the hollow distal end part 22 includes a locking rib that can be locked with the lock adapter 8 at a proximal end part (slightly on a distal end side from the distal end of the hollow main body part 21). In this embodiment, the locking rib is an annular rib.

Furthermore, the lock adapter 8 is a cylindrical member, and is attached to the hollow distal end part 22 of the outer cylinder main body 20. Furthermore, a lower part of the lock adapter 8 is held by the locking rib provided to the hollow distal end part 22. Furthermore, the lock adapter 8 includes an adapter side screw part that can be screwed with a sealing member side screw part of the sealing member (cap) 5 described later. In this first embodiment, the screw part is formed by two spiral ribs.

The sealing member 5 includes a main body part 51 that has a lower hollow part that houses the nozzle part of the hollow distal end part 22, and a seal member 52 that is housed in the main body part 51 and disposed at an upper end part of the lower hollow part. Furthermore, the sealing member side screw part is formed on an outer surface of the lower hollow part.

Examples of materials for forming the outer cylinder main body 20 include various resins such as polypropylene, polyethylene, polystyrene, polyamide, polycarbonate, polyvinyl chloride, poly-(4-methylpentene-1), acrylic resin, acrylonitrile-butadiene-styrene copolymer, polyester such as polyethylene terephthalate, cyclic polyolefin polymer, and cyclic olefin copolymer. Among these resin materials, resins such as polypropylene, cyclic polyolefin polymer, and cyclic olefin copolymer are easy to mold and have heat resistance, and therefore are preferable. Note that the cyclic olefin polymer and the cyclic olefin copolymer that have such high transparency that a drug solution filled inside can be visually checked from an outside, and have heat resistance for withstanding autoclave treatment is particularly preferable as the materials for forming the outer cylinder main body 20.

As a material for forming the lock adapter 8, rigid resins such as polyvinylidene fluoride, polypropylene, polycarbonate, or polyamide are preferably used. As a material for forming the attachment member 60, the sealing member 5, and the plunger 4 described later, rigid or semirigid resins such as high density polyethylene, polypropylene, polystyrene, or polyethylene terephthalate are preferably used.

As a material for forming the seal member 52, elastic materials such as synthetic rubbers such as natural rubber, isoprene rubber, butyl rubber, butadiene rubber, fluororubber, or silicone rubber, and thermoplastic elastomers such as olefin-based elastomer or styrene-based elastomer are preferable.

The gasket 3 includes a gasket main body 31 that includes a closed distal end part 33, an opening rear end part, an inner cavity part that extends from the opening rear end part to a distal end side, and the inner cavity side screw part (a spiral recess part or the plunger distal end part attachment part) 35 that is provided on an inner surface of the inner cavity part.

The gasket 3 is housed in a proximal end part of the outer cylinder main body 20, more specifically, at a position that is located a predetermined length toward the distal end side from a proximal end opening of the outer cylinder main body 20. A distance between a proximal end of the gasket 3 and the proximal end of the outer cylinder main body 20 is preferably 10 to 25 mm.

As illustrated in Figs. 1 and 2, the gasket 3 according to this embodiment includes a cylindrical main body part that extends with substantially the same outer diameter, and a tapered closed part that extends in a distal end direction from the main body part. Furthermore, on an outer side surface of the main body part, a plurality of annular ribs (the number of which is three in this embodiment, yet may be an appropriate number as long as the number is two or more and liquid tightness and slidability can be satisfied) are formed. These ribs come into liquid-tight contact with an inner surface of the outer cylinder main body 20.

Furthermore, the gasket 3 includes a recess part that extends inside the cylindrical main body part and in the distal end direction from the rear end opening part. This recess part can house the gasket attachment part (gasket attachment distal end part) 42 of the plunger 4. Furthermore, on an inner surface of the recess part (an inner surface of the cylindrical main body part), the gasket side screw part (plunger distal end part attachment part) 35 is formed. The gasket side screw part 35 can be screwed with a plunger side screw part formed on the outer surface of the gasket attachment part 42 of the plunger 4. By screwing both of the gasket side screw part 35 and the plunger side screw part, the plunger 4 is not detached from the gasket 3.

As materials for forming the gasket 3, rubbers having elasticity (e.g., butyl rubber, latex rubber, silicone rubber, and the like), synthetic resins (e.g., styrene-based elastomers such as an SBS elastomer and an SEBS elastomer, and olefin-based elastomers such as an ethylene-α-olefin copolymer elastomer), and the like are preferably used.

Furthermore, a distal end side portion of the gasket main body may be coated with a low drug adsorbent substance or the like.

As a material of a low drug adsorbent layer, a known material conventionally used for a laminate gasket can be used. Examples of the material of the low drug adsorbent layer include polyolefin-based resins, fluorine-based resins, and polyester-based resins. More specifically, the polyolefin-based resins are preferably polypropylene, ultra-high molecular weight polyethylene, poly(4-methylpentene-1), cyclic polyolefin, and the like, and the fluorine-based resins are preferably tetrafluoroethylene-perfluoroethoxyethylene copolymer, polytetrafluoroethylene, tetrafluoroethylene/perfluoroalkyl vinyl ether copolymer, tetrafluoroethylene/hexafluoropropylene copolymer, and the like.

Furthermore, it is preferable to apply a lubricant to the outer surface of the gasket main body 31, that is, at least the surfaces of a distal end side annular rib 36 and a rear end side annular rib 37. Furthermore, the lubricant may be applied to the inner surface of the outer cylinder. As the lubricant, silicone oil is suitable. Furthermore, the surface of the gasket main body may be provided with a silicone-based resin layer solidified and formed on the surface, or a layer made of the above-described ultra-high molecular weight polyethylene or the fluorine-based resin so as not to use a lubricant such as silicone oil.

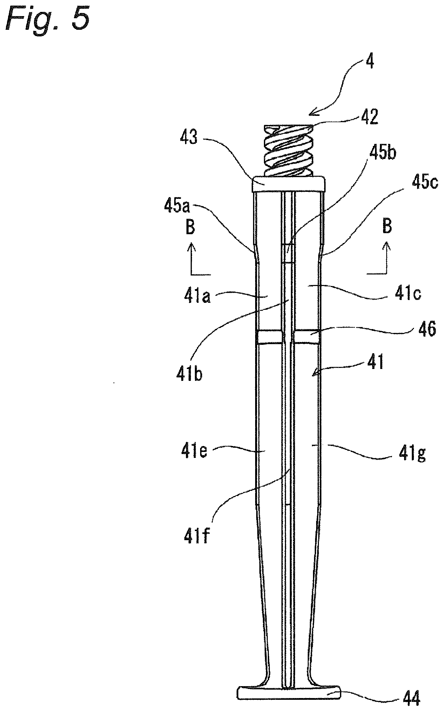

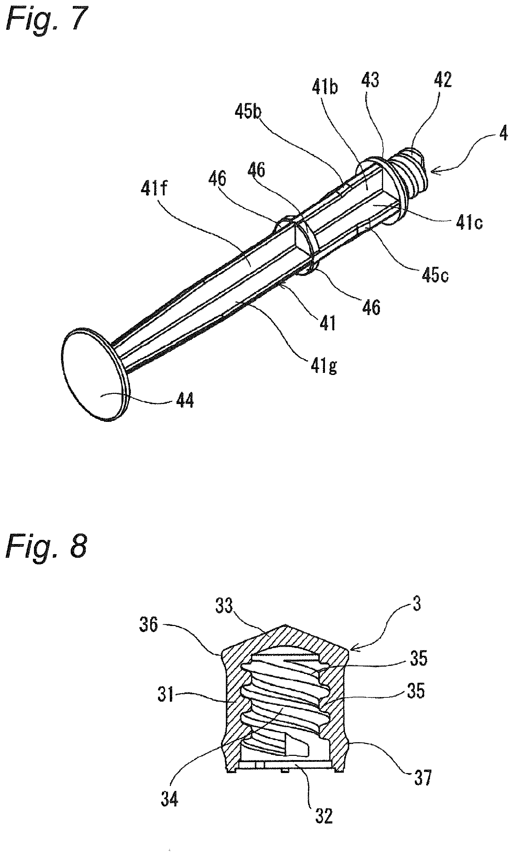

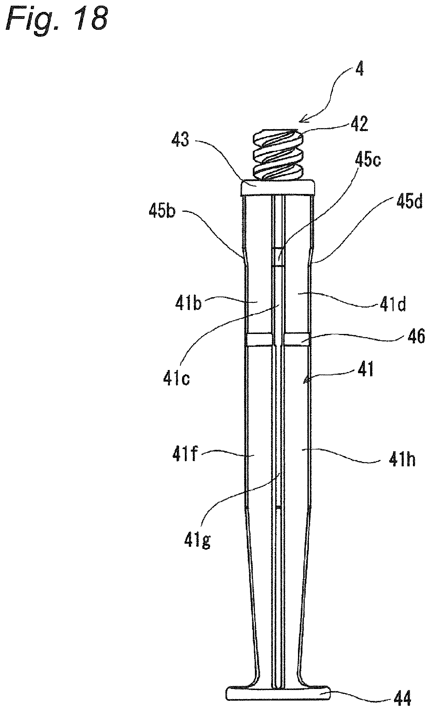

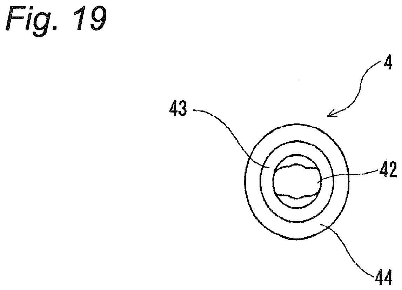

As illustrated in Figs. 1 to 7, 18, and 19, the plunger (syringe plunger) 4 includes the gasket attachment part 42 that is provided at an upper end, the gasket pressing part 43 that is located at a lower end of the gasket attachment part 42, the shaft part 41 that extends downward from the gasket pressing part 43, and the pressing operation part 44 that is provided near the rear end of the shaft part 41.

The gasket attachment part 42 is a protruding part that is provided at a distal end part of the plunger 4, and can enter an inner cavity part 34 of the gasket 3. The gasket attachment part 42 protrudes in the distal end direction from a vicinity of a center of the disc-shaped gasket pressing part 43. The gasket attachment part 42 is desirably a cylindrical part, yet may be a rod-like part. Furthermore, the gasket attachment part 42 may be a cylindrical part that includes a cross-shaped reinforcing part inside.

Furthermore, the outer surface of the gasket attachment part 42 includes the plunger side screw part that is screwed with the gasket side screw part 35 formed on the inner surface of the inner cavity part 34 of the gasket 3. The plunger side screw part is formed by a spiral rib. In this embodiment, the plunger side screw part includes two strips (two rows) of spiral ribs so as to match the spiral screw part of the gasket 3. The spiral rib may be only one strip (one row). Furthermore, in the prefilled syringe according to this embodiment, both of the gasket 3 and the plunger 4 are placed in the attached state by rotating the plunger 4.

The gasket pressing part 43 is a plate-like part that has a substantially perfect circular shape, and has a diameter that is slightly smaller than the inner diameter of the outer cylinder main body 20. The gasket attachment part 42 is provided such that the center of the gasket attachment part 42 is located on a center axis of the gasket pressing part 43.

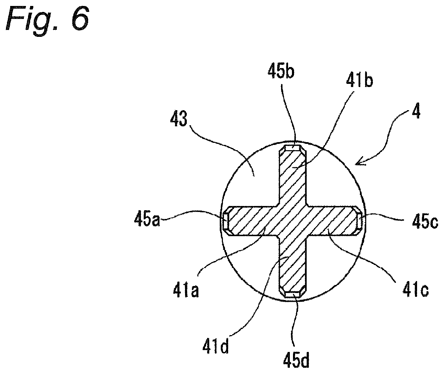

The shaft part 41 is formed by three or more (four in this embodiment) flat plate parts extending from the gasket pressing part 43 to a rear end direction side and in parallel to a center axis of the plunger 4. As illustrated in Figs. 1 to 7, 18, and 19, and Fig. 6 in particular, the shaft part 41 according to this embodiment is formed by the four flat plate parts extending radially at equal angles (more specifically, at intervals of 90 degrees,) from a center axis of the shaft part 41. Furthermore, as illustrated in Fig. 6, a bonding part of each flat plate part of the shaft part 41 according to this embodiment is provided with an axial direction reinforcing part that extends over the entire length of the flat plate part. Therefore, the shaft part 41 has a thick center part, in other words, has a thick shaft axis.

The pressing operation part 44 is provided at the rear end of the shaft part 41. The pressing operation part 44 is formed in a disk shape, and is used as a plunger pressing part. The pressing operation part 44 according to this embodiment has a disk shape that has a larger diameter than that of the above-described disc-shaped gasket pressing part 43.

Furthermore, according to this embodiment, there are disc-shaped, in other words, four 1/4 circular reinforcing parts 46 located in the middle of the shaft part 41, more specifically, on a distal end side from the center. Therefore, each flat plate part includes distal end side flat plate parts 41a, 41b, 41c, and 41d, and rear end side flat plate parts 41e, 41f, 41g, and 41h. Furthermore, the distal end side flat plate part 41a and the rear end side flat plate part 41e are aligned on the same straight line. Similarly, the distal end side flat plate part 41b and the rear end side flat plate part 41f, the distal end side flat plate part 41c and the rear end side flat plate part 41g, and the distal end side flat plate part 41d and the rear end side flat plate part 41h are also aligned on the same straight line.

In other words, the reinforcing parts 46 partition each flat plate part into the distal end side flat plate parts 41a, 41b, 41c, and 41d and the rear end side flat plate parts 41e, 41f, 41g, and 41h. The rear end side flat plate parts 41e, 41f, 41g, and 41h extend from the rear ends (reinforcing parts 46) of the distal end side flat plate parts 41a, 41b, 41c, and 41d to the pressing operation part 44 in parallel to the center axis of the plunger. Note that, according to this embodiment, the distal end side flat plate parts 41a, 41b, 41c, and 41d are thicker than the rear end side flat plate parts 41e, 41f, 41g, and 41h.

Furthermore, the distal end side flat plate parts 41a, 41b, 41c, and 41d have a predetermined thickness that is relatively thick, and extend toward the rear end direction side from the gasket pressing part 43 and in parallel to the center axis of the plunger 4.

Furthermore, the shaft part 41 includes indicator parts 45a, 45b, 45c, and 45d that are located on the rear end side of the gasket pressing part 43, are located on the distal end side of the reinforcing parts 46, and can be recognized by visually checking an external appearance.

The indicator part can be formed by a unique outer shape part that has a different shape from that of an other portion of the shaft part (more specifically, the flat plate part) and therefore is visually recognizable, a unique outer surface part that has a different surface state from that of the other portion of the shaft part (more specifically, the flat plate part) and therefore is visually recognizable, and the like. Furthermore, the indicator part may be formed by a unique outer shape part and a unique outer surface part that have a different surface state and shape from those of the other portion of the shaft part.

In this embodiment, the indicator parts 45a, 45b, 45c, and 45d are formed by inclined parts that are formed on an outer surface of the shaft part, more specifically, outer side surfaces of the distal end side flat plate parts 41a, 41b, 41c, and 41d. Furthermore, in this embodiment, the indicator parts (unique outer shape parts) 45a, 45b, 45c, and 45d include vertex plane parts that extend in an axial direction of the plunger 4, and inclined side surface parts that are provided on both sides of the vertex plane parts.

Therefore, the indicator parts 45a, 45b, 45c, and 45d are easily visually recognized. Note that, in this embodiment, the distal end side flat plate parts 41a, 41b, 41c, and 41d each include in the entire length thereof the vertex plane part, and the inclined side surface part that are provided on both sides of the vertex plane parts. Note that only the indicator parts 45a, 45b, 45c, and 45d portions may each include the vertex plane part that extends in the axial direction of the plunger 4, and the inclined side surface part that is provided on the both sides of the vertex plane part.

Furthermore, inclination angles of the indicator parts 45a, 45b, 45c, and 45d with respect to the center axis of the plunger 4 are preferably 6 to 27 degrees, and are particularly preferably 9 to 22 degrees. Furthermore, the length of each indicator part in the axial direction of the plunger is preferably 1 to 6 mm, and is particularly preferably 2 to 6 mm. Furthermore, the length of the indicator part in the axial direction of the plunger is preferably an allowable plunger moving distance, in other words, a moving distance of the plunger that does not cause an influence in keeping a sterilized state.

In the prefilled syringe 1 according to the present invention, when the gasket unnecessarily moves by a time of an administration operation, the indicator parts 45a, 45b, 45c, and 45d are located behind the flange part 6, and can be visually recognized behind the flange part 6. Consequently, a person who performs the administration operation can grasp the probability of movement of the gasket 3. Particularly when the entire indicator parts 45a, 45b, 45c, and 45d are located behind the flange part 6, and are visually recognizable, there is a probability that a movement amount of the gasket is large, air flows from the side surface of the gasket 3 into the outer cylinder 2, and the sterilized state is not kept, and it is possible to easily grasp such a probability.

Note that, in the prefilled syringe 1 according to this embodiment, the plunger 4 and the gasket 3 are coupled by screwing. There is also a probability that loosening of attachment (more specifically, loosening of screwing) of the plunger 4 to the gasket 3 makes the plunger move backward. Therefore, when the indicator parts 45a, 45b, 45c, and 45d are housed in the flange part 6 by rotating the plunger 4, it is possible to check that the gasket 3 has not unnecessarily moved backward.

Furthermore, in the prefilled syringe 1 according to this embodiment, the plunger 4 is attached to the gasket 3, and the indicator parts 45a, 45b, 45c, and 45d are entirely located in the flange part 6 of the outer cylinder 2 in a state where the plunger 4 has been attached to the gasket 3. Furthermore, the rear ends of the indicator parts 45a, 45b, 45c, and 45d are located at the same positions as or slightly on an inner side of a rear end of the flange part 6 of the outer cylinder 2.

Furthermore, in the prefilled syringe 1 of this embodiment, as described above, the flange part 6 is formed by the attachment member 60 fixed to the outer cylinder flange 24, the attachment member 60 includes the plunger insertion cutout part 63 that extends from the side part to the center part of the attachment member 60, and, instead of all the indicator parts, part of all the indicator parts, more specifically, the indicator part 45d that is part of the indicator parts is visually recognizable through the plunger insertion cutout part 63. However, the rest of the indicator parts 45a, 45b, and 45c cannot be visually recognized. Consequently, the visually recognizable indicator part 45d makes it possible to recognize the presence of the indicator part, and the rest of the indicator parts 45a, 45b, and 45c cannot visually checked, so that it is possible to easily recognize that the gasket does not unnecessarily move.

Furthermore, the prefilled syringe 1 according to this embodiment further includes a label 27 that is pasted on an outer surface of the outer cylinder 2, and the gasket 3 cannot be visually recognized due to the label 27. Therefore, according to this prefilled syringe 1, whether or not the gasket moves cannot be visually recognized. However, the prefilled syringe 1 according to this embodiment includes the above-described indicator parts, so that, even when the gasket 3 itself cannot be visually recognized, it is possible to visually recognize unnecessary backward movement of the gasket 3.

The drug solution 9 to be filled in a space formed by the outer cylinder main body 20, the gasket 3, and the sealing member 5 may be any drug and, for example, a drug solution such as cyclosporine, a benzodiazepine-based drug, a sodium chloride injection solution, vitamins, or minerals, a powdery or lyophilized drug such as an antibiotic or a protein preparation, or a solution is used.

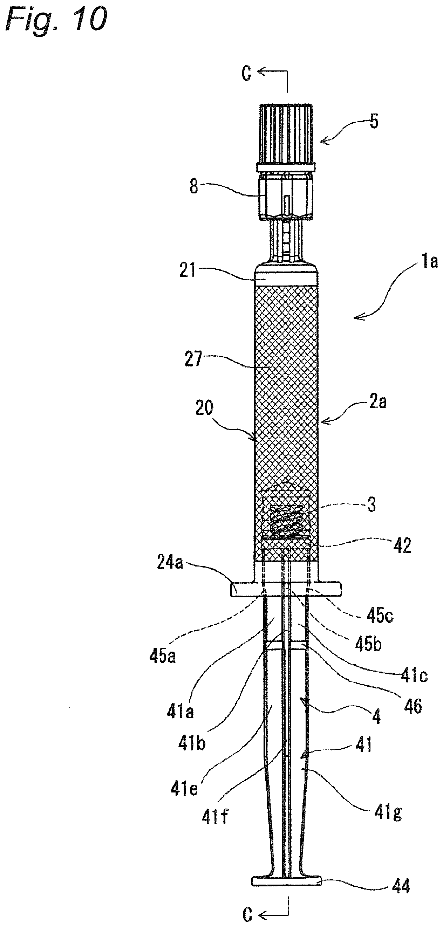

Furthermore, the prefilled syringe according to the present invention may be a prefilled syringe 1a illustrated in Figs. 10 and 11 in which an outer cylinder 2a does not include an attachment member. In the prefilled syringe 1a according to this embodiment, a flange part is formed by a flange 24a of the outer cylinder 2a. In addition, Fig. 11 illustrates cross sections of the outer cylinder and a sealing member taken along line C-C in Fig. 10, and illustrates external appearances of a plunger and a gasket.

Only differences between the prefilled syringe 1a according to this embodiment and the above-described prefilled syringe 1 are that whether or not there is the attachment member, and the gasket 3 and the plunger 4 are located slightly on the distal end side of the outer cylinder. The rest is the same as those of the above-described prefilled syringe 1.

Furthermore, in the prefilled syringe 1a according to this embodiment, too, the plunger 4 is attached to the gasket 3, and the indicator parts 45a, 45b, 45c, and 45d are entirely located in the flange 24a of the outer cylinder 2a in a state where the plunger 4 has been attached to the gasket 3. Furthermore, the rear ends of the indicator parts 45a, 45b, 45c, and 45d are located at the same positions as or slightly on an inner side of a rear end of the flange 24a of the outer cylinder 2a. Furthermore, all the indicator parts 45a, 45b, 45c, and 45d cannot be visually recognized due to the flange 24a of the outer cylinder 2a.

Note that, in the prefilled syringe 1a according to this embodiment, distal ends or distal end parts of the indicator parts 45a, 45b, 45c, and 45d may be visually recognizable from the outer surface of the outer cylinder 2a at the proximal end part of the hollow main body part 21 (a boundary part with the flange 24a) of the outer cylinder 2a. Such a prefilled syringe makes it possible to recognize presence of the indicator parts, and makes it possible to easily recognize that the gasket does not unnecessarily move since parts other than the distal end parts of the indicator parts 45a, 45b, 45c, and 45d cannot be visually recognized.

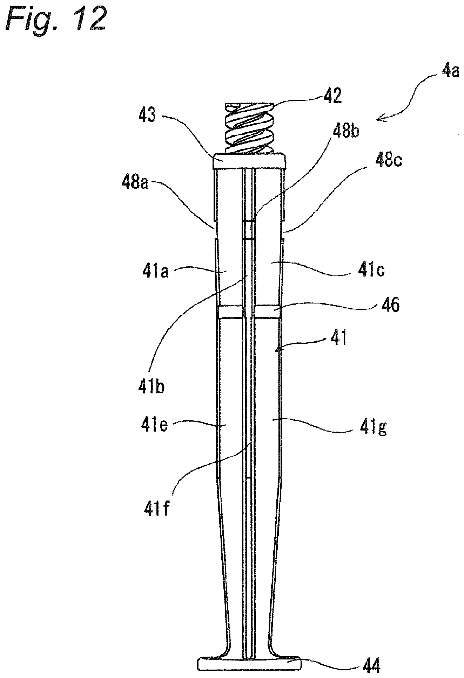

Furthermore, an aspect of the indicator parts in the prefilled syringe according to the present invention may include indicator parts included in a plunger 4a illustrated in Fig. 12.

Similarly to the above-described indicator parts 45a, 45b, 45c, and 45d, indicator parts 48a, 48b, 48c, and 48d (not illustrated) included in the plunger 4a according to this embodiment are each a unique outer shape part that is visually recognizable since each indicator part has a different shape from that of the other portion of the shaft part (more specifically, the flat plate part).

Furthermore, in this embodiment, the indicator parts (unique outer shape parts) 48a, 48b, 48c, and 48d (not illustrated) are each formed by a recess part that extends a predetermined length from a vertex of each flat plate part toward a proximal end side of the flat plate part, and the recess part in particular has a rectangular shape. The depth of the recess part is preferably 0.3 to 0.6 mm, and is particularly preferably 0.4 to 0.5 mm.

Furthermore, the indicator parts 48a, 48b, 48c, and 48d (not illustrated) portions are slightly inclined parts, yet are considerably gentler than the inclination angles of the above-described indicator parts 45a, 45b, 45c, and 45d, and the recess parts are more noticeable than the inclinations.

Only differences between the plunger 4a according to this embodiment and the above-described plunger 4 are the above-described forms of the indicator parts and the shapes of the distal end side flat plate parts 41a, 41b, 41c, and 41d on the proximal end side of the indicator parts.

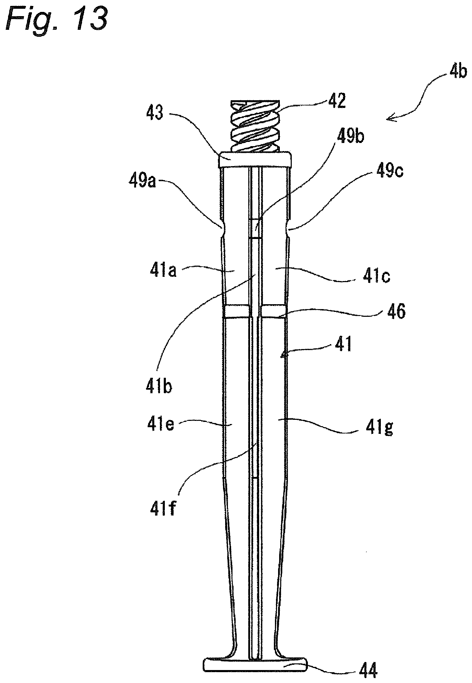

Furthermore, an aspect of the indicator parts in the prefilled syringe according to the present invention may include indicator parts included in a plunger 4b illustrated in Fig. 13.

Similarly to the above-described indicator parts 45a, 45b, 45c, and 45d, indicator parts 49a, 49b, 49c, and 49d (not illustrated) included in the plunger 4b according to this embodiment are each a unique outer shape part that is visually recognizable since each indicator part has a different shape from that of the other portion of the shaft part (more specifically, the flat plate part).

Furthermore, in this embodiment, the indicator parts (unique outer shape parts) 49a, 49b, 49c, and 49d (not illustrated) are each formed by a recess part that extends a predetermined length from the vertex of each flat plate part toward a proximal end side of the flat plate part, and the recess part in particular is a recess part whose lower surface is an arc shape. The depth of the recess part is preferably 0.3 to 0.6 mm, and is particularly preferably 0.4 to 0.5 mm.

Furthermore, the indicator parts 49a, 49b, 49c, and 49d (not illustrated) portions are slightly inclined parts, yet are considerably gentler than the inclination angles of the above-described indicator parts 45a, 45b, 45c, and 45d, and the recess parts are more noticeable than the inclinations.

Only differences between the plunger 4b according to this embodiment and the above-described plunger 4 are the above-described forms of the indicator parts and the shapes of the distal end side flat plate parts 41a, 41b, 41c, and 41d on the proximal end side of the indicator parts.

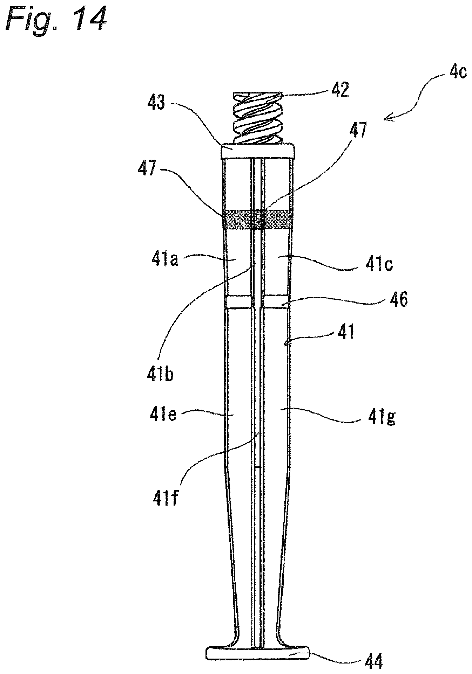

Furthermore, an aspect of the indicator part in the prefilled syringe according to the present invention may include an indicator part included in a plunger 4c illustrated in Fig. 14.

An indicator part 47 included in the plunger 4c according to this embodiment is formed by a unique outer surface part that has a different surface state from that of the other portion of the shaft part and therefore is visually recognizable. The indicator part 47 (unique outer surface part) can be formed by a belt-shaped colored part, a rough surface part, a patterned part, or the like that is formed on the outer surface of the shaft part. The width (an axial direction length of the plunger 4c) of the belt-shaped indicator part 47 (unique outer surface part) is preferably 1 to 6 mm, and is particularly preferably 2 to 6 mm.

For the colored part, a color that is an easily identifiable color and can be recognized as a warning indication is preferable, and, for example, red colors are preferable, but yellow, blue, or the like may be used. The rough surface part may be an embossed surface, a knurled surface, a surface having a large number of ribs, or the like. For the patterned surface, various patterns such as a checkered pattern, a wave pattern, and the like can be used.

Only differences between the plunger 4c according to this embodiment and the above-described plunger 4 are the above-described form of the indicator part and the shapes of the distal end side flat plate parts 41a, 41b, 41c, and 41d on the proximal end side of the indicator part.

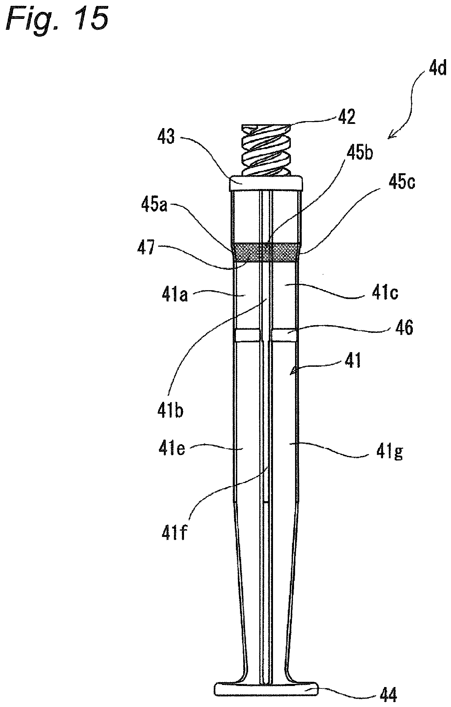

Furthermore, an aspect of the indicator parts in the prefilled syringe according to the present invention may include indicator parts included in a plunger 4d illustrated in Fig. 15.

The indicator part 47 included in the plunger 4d according to this embodiment is formed by both of a unique outer shape part that has a different shape from that of the other portion of the shaft part and therefore is visually recognizable, and a unique outer surface part that has a different surface state and therefore is visually recognizable. As the indicator part 47, the above-described indicator part can be suitably used.

The plunger 4d according to this embodiment includes the same indicator parts (unique outer shape parts) 45a, 45b, 45c, and 45d as those of the above-described plunger 4, and includes the belt-shaped indicator part 47 (unique outer surface part) included in the above-described plunger 4c at parts where the indicator parts (unique outer shape parts) 45a, 45b, 45c, and 45d are provided. Consequently, it is easier to recognize the indicator parts.

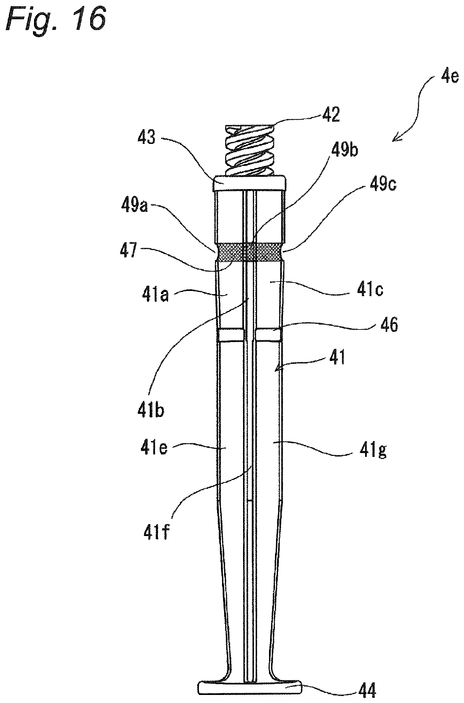

Furthermore, an aspect of the indicator parts in the prefilled syringe according to the present invention may include indicator parts included in a plunger 4e illustrated in Fig. 16.

The indicator part 47 included in the plunger 4e according to this embodiment is formed by both of a unique outer shape part that has a different shape from that of the other portion of the shaft part and therefore is visually recognizable, and a unique outer surface part that has a different surface state and therefore is visually recognizable. As the indicator part 47, the above-described indicator part can be suitably used.

The plunger 4e according to this embodiment includes the same indicator parts (unique outer shape parts) 49a, 49b, 49c, and 49d (not illustrated) as those of the above-described plunger 4b. The indicator parts (unique outer shape parts) 49a, 49b, 49c, and 49d (not illustrated) are each formed by a recess part that extends a predetermined length from the vertex of each flat plate part toward a proximal end side of the flat plate part, and the recess part in particular is a recess part whose lower surface is an arc shape. Note that the recess part may have a rectangular shape. Furthermore, the indicator parts 49a, 49b, 49c, and 49d (not illustrated) portions are slightly inclined parts, yet are considerably gentler than the inclination angles of the above-described indicator parts 45a, 45b, 45c, and 45d, and the recess parts are more noticeable than the inclinations.

The plunger 4e according to this embodiment includes the same indicator parts (unique outer shape parts) 49a, 49b, 49c, and 49d (not illustrated) as those of the above-described plunger 4b, and includes the belt-shaped indicator part 47 (unique outer surface part) included in the above-described plunger 4c at parts where the indicator parts (unique outer shape parts) 49a, 49b, 49c, and 49d (not illustrated) are provided. Consequently, it is easier to recognize the indicator parts.

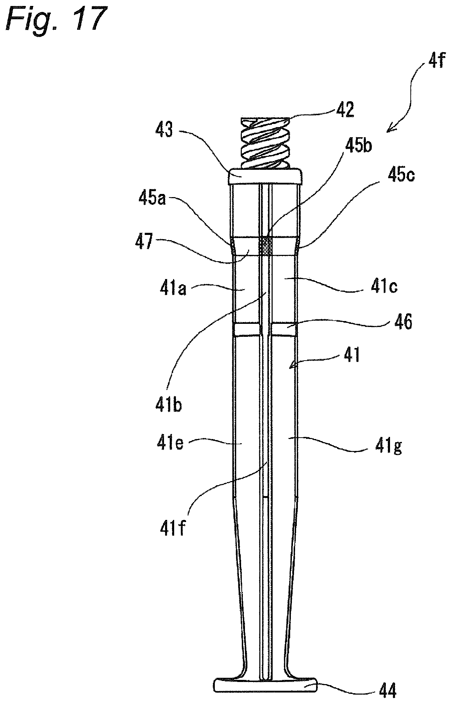

Furthermore, an aspect of the indicator parts in the prefilled syringe according to the present invention may include indicator parts included in a plunger 4f illustrated in Fig. 17.

The indicator part 47 included in the plunger 4f according to this embodiment is formed by both of a unique outer shape part that has a different shape from that of the other portion of the shaft part and therefore is visually recognizable, and a unique outer surface part that has a different surface state and therefore is visually recognizable. As the indicator part 47, the above-described indicator part can be suitably used.

The plunger 4f according to this embodiment includes the same indicator parts (unique outer shape parts) 45a, 45b, 45c, and 45d (not illustrated) as those of the above-described plunger 4. Furthermore, upper surfaces of the indicator parts (unique outer shape parts) 45a, 45b, 45c, and 45d (not illustrated) are unique outer surface parts similar to the indicator part 47. Consequently, it is easier to recognize the indicator parts. For the unique outer surface parts, all the above-described patterns such as the colored part, the rough surface part, and the patterned part can be used.

### Industrial Applicability

The prefilled syringe according to the present invention is as follows.
(1) A prefilled syringe including: an outer cylinder; a gasket that is slidably housed in the outer cylinder; a plunger that presses the gasket; a sealing member that seals a distal end part of the outer cylinder; and a drug that is housed in the outer cylinder, in which the outer cylinder includes a flange part that is provided at a rear end part, the gasket includes a plunger distal end part attachment part, the plunger includes a gasket pressing part that is provided at a distal end of the plunger, a shaft part that extends in a rear end direction from the gasket pressing part, and a pressing operation part that is provided at a rear end part of the shaft part, the shaft part includes on a rear end side of the gasket pressing part an indicator part that can be recognized by visually checking an external appearance, the plunger is attached to the gasket, and the indicator part is located in the flange part of the outer cylinder, and cannot be recognized by visually checking the external appearance in a state where the plunger has been attached to the gasket, and is exposed behind the flange part, and can be recognized by visually checking the external appearance when the plunger moves backward.
   According to this prefilled syringe, when the plunger attached to the gasket moves backward beyond an allowable level, the indicator parts located in the flange part of the outer cylinder are exposed to the outside from the rear end of the flange part of the outer cylinder and can be easily visually recognized. Consequently, by visually recognizing the indicator parts, the user can easily recognize a probability that the gasket has moved beyond the allowable level and air has flowed into the drug, in other words, a probability that the drug has been contaminated.
   Furthermore, the above embodiment may be as follows.
(2) The prefilled syringe according to above (1), includ'ng a label that is pasted on an outer surface of the outer cylinder, in which the gasket cannot be visually recognized due to the label.
(3) The prefilled syringe according to above (1) or (2), in which the flange part of the outer cylinder is formed by an outer cylinder flange that is provided at a rear end part of the hollow main body part, and an attachment member that is attached to a rear end side of the outer cylinder flange.
(4) The prefilled syringe according to above (3), in which the attachment member includes a plunger insertion cutout part that extends from a side part to a center part of the attachment member, and part of the indicator part is visually recognizable through the plunger insertion cutout part.
(5) The prefilled syringe according to any one of above (1) to (4), in which a length of the indicator part in an axial direction of the plunger is 1 to 6 mm.
(6) The prefilled syringe according to any one of above (1) to (5), in which the indicator part is formed by a unique outer shape part that has a different shape from a shape of an other portion of the shaft part and is visually recognizable.
(7) The prefilled syringe according to any one of above (1) to (5), in which the indicator part is formed by a unique outer surface part that has a different surface state from a surface state of an other portion of the shaft part and is visually recognizable.
(8) The prefilled syringe according to any one of above (1) to (6), in which the indicator part is an inclined part, a recess part, a protrusion part, or a tapered part formed on an outer surface of the shaft part.
(9) The prefilled syringe according to any one of above (1) to (5), in which the indicator part is a colored part, a rough surface part, or a patterned part formed on an outer surface of the shaft part.
(10) The prefilled syringe according to any one of above (1) to (9), in which the indicator part is formed by a visually recognizable different shape/state part that has a different surface state and shape from a surface state and a shape of an other portion of the shaft part.

## Claims

1. A prefilled syringe comprising: an outer cylinder; a gasket that is slidably housed in the outer cylinder; a plunger that presses the gasket; a sealing member that seals a distal end part of the outer cylinder; and a drug that is housed in the outer cylinder, wherein
the outer cylinder includes a flange part that is provided at a rear end part,
the gasket includes a plunger distal end part attachment part,
the plunger includes a gasket pressing part that is provided at a distal end of the plunger, a shaft part that extends in a rear end direction from the gasket pressing part, and a pressing operation part that is provided at a rear end part of the shaft part,
the shaft part includes on a rear end side of the gasket pressing part an indicator part that can be recognized by visually checking an external appearance, the plunger is attached to the gasket, and the indicator part is located in the flange part of the outer cylinder, and cannot be recognized by visually checking the external appearance in a state where the plunger has been attached to the gasket, and is exposed behind the flange part, and can be recognized by visually checking the external appearance when the plunger moves backward.

2. The prefilled syringe according to claim 1, comprising a label that is pasted on an outer surface of the outer cylinder, wherein the gasket cannot be visually recognized due to the label.

3. The prefilled syringe according to claim 1 or 2, wherein the flange part of the outer cylinder is formed by an outer cylinder flange that is provided at a rear end part of the hollow main body part, and an attachment member that is attached to a rear end side of the outer cylinder flange.

4. The prefilled syringe according to claim 3, wherein the attachment member includes a plunger insertion cutout part that extends from a side part to a center part of the attachment member, and part of the indicator part is visually recognizable through the plunger insertion cutout part.

5. The prefilled syringe according to any one of claims 1 to 4, wherein a length of the indicator part in an axial direction of the plunger is 1 to 6 mm.

6. The prefilled syringe according to any one of claims 1 to 5, wherein the indicator part is formed by a unique outer shape part that has a different shape from a shape of an other portion of the shaft part and is visually recognizable.

7. The prefilled syringe according to any one of claims 1 to 5, wherein the indicator part is formed by a unique outer surface part that has a different surface state from a surface state of an other portion of the shaft part and is visually recognizable.

8. The prefilled syringe according to any one of claims 1 to 6, wherein the indicator part is an inclined part, a recess part, a protrusion part, or a tapered part that is formed on an outer surface of the shaft part.

9. The prefilled syringe according to any one of claims 1 to 5, wherein the indicator part is a colored part, a rough surface part, or a patterned part that is formed on an outer surface of the shaft part.

10. The prefilled syringe according to any one of claims 1 to 9, wherein the indicator part is formed by a visually recognizable different shape/state part that has a different surface state and shape from a surface state and a shape of an other portion of the shaft part.
